# EUROPÄISCHE PATENTANMELDUNG

(11) **EP 0 943 333 A1**
(43) Veröffentlichungstag der Anmeldung: **22.09.1999**
(21) Anmeldenummer: 98104949.7
(22) Anmeldetag: 18.03.1998
(51) Int. Cl.: A61K 31/565

(54) **Medikament zur Prophylaxe und/oder Behandlung des Mammakarzinoms enthaltend einen Hemmer der Bildung von Oestrogenen**

(71) Anmelder: S.W. Patentverwertungs GmbH, 5026 Salzburg (AT)
(72) Erfinder: Schmidt, Alfred, Dr., D-22529 Hamburg (DE); Wieland, Heinrich, Prof. Dr., 79271 St. Peter (DE)
(74) Vertreter: Glawe, Delfs, Moll & Partner

(57) **Zusammenfassung**

Gegenstand der Erfindung ist die Verwendung einer oder mehrerer die Bildung von Östrogenen inhibierenden Substanzen zur Herstellung eines für die topische Applikation formulierten Medikaments zur Prophylaxe und/oder Behandlung des Mammakarzinoms. Das erfindungsgemäße Medikament vermeidet bei systemischer Anwendung auftretende Nebenwirkungen und erlaubt daher die Durchführung einer Primärprophylaxe oder aber nach Auftreten eines Mammakarzinoms die Durchführung einer Sekundärprophylaxe.

## Beschreibung

Die Erfindung betrifft ein Medikament zur Prophylaxe (Primär-und Sekundärprophylaxe) und/oder Behandlung des Mammakarzinoms.

Das Mammakarzinom (Brustkrebs) ist bei Frauen die häufigste maligne Erkrankung. In Deutschland macht der Brustkrebs etwa 20% aller Krebsfälle bei Frauen aus, die Inzidenz liegt derzeit bei über 30.000 Neuerkrankungen pro Jahr. Die heute angewendete adjuvante Krebstherapie führt zwar zu einer Steigerung der Überlebensrate, auch kann Brustkrebsscreening und frühe chirurgische Behandlung die Mortalität um über 30% senken. Da jedoch die Anzahl der Neuerkrankungen ständig zunimmt, bleibt die Todesrate gemessen an der Gesamtbevölkerung gleich oder steigt. Die Zahl der Neuerkrankungen läßt sich bisher kaum beeinflussen, da zuwenig über auslösende Faktoren bekannt ist.

Bei etwa der Hälfte aller Mammakarzinome finden sich Östrogen- und/oder Progesteronrezeptoren im Zytoplasma. Solche Mammakarzinome benötigen für die Proliferation ihrer Zellen Östrogen. Östrogene wirken durch Bindung an spezifische intrazelluläre (zytoplasmatische) Rezeptoren von östrogenempfindlichen Zellen, in die sie passiv durch Diffusion aus dem Plasma eingeschleust werden. Die Bindung verändert die Konfiguration des Rezeptorproteins. Der Rezeptor-Hormon-Komplex steuert sowohl die Transkription als auch die Expression spezifischer Gene, die dadurch bewirkte Synthese von wachstumsfördernden und/oder wachstumshemmenden Faktoren wirkt letztlich auf das Zellwachstum ein.

Durch Entzug von Östrogenen kann eine Rückbildung östrogenabhängiger Tumoren bewirkt werden. Bei prämenopausalen Frauen sind die Ovarien Hauptquelle von Östrogenen. Deren chirurgische Entfernung wird daher bereits seit 1896 bei Brustkrebs im fortgeschrittenen Stadium (Metastasenbildung) als sogenannte chirurgische Hormontherapie durchgeführt.

Bei postmenopausalen Frauen ist die Umwandlung adrenaler Androgene, vor allem Androstendion und Testosteron, zu Östron und Östradiol die Hauptöstrogenquelle. Die Umwandlung zu Östrogenen findet im Muskel- und Fettgewebe statt.

In der klinischen Praxis werden seit über zwanzig Jahren sowohl frühe als auch fortgeschrittene Stadien des Mammakarzinoms mit Tamoxifen oder dessen Derivaten (insbesondere Tamoxifencitrat) behandelt. Tamoxifen belegt die im Zytoplasma der Krebszellen vorhandenen Östrogenrezeptoren und bewirkt so eine kompetitive Verdrängung von Östrogenen. Der aus Tamoxifen und dem Östrogenrezeptor gebildete Komplex verhindert die ansonsten von einem Komplex aus Östrogenen und dem Rezeptor bewirkte Transkription und Expression der das Zellwachstum fördernden Gene.

Bei in vitro-Versuchen ist nachgewiesen worden, daß Tamoxifen auch auf Zellinien wachstumshemmend und unter Umständen sogar zytostatisch wirkt, die über keine Östrogenrezeptoren verfügen. Tamoxifen hemmt die Proteinkinase C und blockiert die Aktivierung von Calmodulin. Es steigert die Aktivität der Killerzellen und hemmt Suppressor T-Lymphozyten.

Insbesondere nach längerer Behandlung kann Tamoxifen auf eine nicht näher bekannte Art und Weise auf Krebszellen auch wie Östrogene wirken und deren Wachstum fördern. Eine länger andauernde Tamoxifen-Behandlung kann daher u.U. zu einem Tumorwachstum führen. Sie führt zudem zu einem um den Faktor 3 bis 5 erhöhten Risiko, an Krebs der Gebärmutterschleimhaut zu erkranken. Angesichts des klinischen Nutzens der Tamoxifen-Therapie nimmt man dieses Risiko bei Brustkrebspatientinnen in Kauf.

Bekannt ist ferner die systemische Behandlung von Mammakarzinomen mit Aromataseinhibitoren, insbesondere 4-Hydroxyandrost-4-en-3,17-dion (INN Formestan). Die Aromatase ist ein komplexes Enzymsystem, das die Umwandlung adrenaler Androgene zu Östron und Östradiol katalysiert.

Formestan ist ein Derivat des physiologischen Stereoidhormons Androstendion und bindet kompetetiv zu anderen Substraten an Aromatase. Es schädigt während der Katalyse das Enzymmolekül irreversibel. Die systemische Behandlung mit Formestan wird ebenfalls als antiöstrogene Brustkrebstherapie angewandt.

Der Erfindung liegt die Aufgabe zugrunde, ein Medikament der eingangs genannten Art zu schaffen, das für eine Behandlung und insbesondere auch Prophylaxe des Mammakarzinoms geeignet ist.

Die Erfindung löst diese Aufgabe dadurch, daß eine oder mehrere die Bildung von Östrogenen inhibierende Substanzen zu einem für die topische Applikation vorgesehenen Medikament formuliert werden.

Zunächst seien einige im Rahmen der Erfindung verwendete Begriffe erläutert.

Unter "Östrogenen" sind alle weiblichen Sexualhormone mit einer Wirkung vergleichbar bspw. der von Östron und Östradiol zu verstehen.

Erfindungsgemäß werden die Bildung von Östrogenen inhibierende Substanzen verwendet. Diese inhibieren die Östrogenbiosynthese aus den androgenen Vorstufen, bspw. die enzymatische Umwandlung von Androstendion zu Östron oder von Testosteron zu Östradiol. Da die beiden genannten Syntheseschritte durch das Enzymsystem der Aromatase (converting enzyme) vermittelt werden, sind Aromataseinhibitoren im Rahmen der Erfindung besonders geeignet. Es kann sich dabei um steroidale und nichtsteroidale Inhibitoren handeln. Bevorzugt sind Inhibitoren, die an die Aromatase binden und diese irreversibel schädigen. Nach der topischen Applikation penetrieren sie durch die Haut und reichern sich im Fettgewebe an.

Das erfindungsgemäße Medikament ist für die topische Applikation vorgesehen. Das Medikament wird lokal auf die Haut aufgetragen, der vorzugsweise stark lipophile Wirkstoff wird transdermal resorbiert und somit lokal an den vorgesehenen Wirkort gebracht. Der Wirkstoff reichert sich im periduktalen Fettgewebe an. Bei einer Langfristbehandlung wird die Fettmasse der behandelten Brust deutlich reduziert. Diese Reduktion vermindert die Menge der Östrogenbildnerzellen mit Östrogenbildner-Kompetenz. Die Lipophilie und Hydrophobie des Wirkstoffs hat zur Folge, daß sich der Wirkstoff ausschließlich lokal im Fettgewebe anreichert und keine systemische Wirkung entfalten kann.

Das Medikament kann zur Behandlung des Mammakarzinoms dienen. Diese Behandlung kann nach einer chirurgischen Primärversorgung und ggf. entsprechender adjuvanter Therapie die bisher übliche systemische antiöstrogene Therapie ersetzen und/oder ergänzen.

Ein wichtiger Vorteil der Erfindung ist die Möglichkeit, das Medikament auch für die Brustkrebsprophylaxe einzusetzen. Eine besonders vorteilhafte Einsatzmöglichkeit ist die sogenannte Sekundärprophylaxe. Bei Patientinnen, bei denen bereits ein Mammakarzinom vorliegt, besteht ein besonders hohes Risiko für ein weiteres Karzinom in der kontralateralen Brust. Die kontralaterale Brust kann dann mit dem erfindungsgemäßen Medikament prophylaktisch behandelt werden. Ebenfalls möglich ist eine sekundärprophylaktische Behandlung der erkrankten Brust zur Vermeidung lokaler Rezidive.

Bei sogenannten Hochrisikofrauen kann eine Primärprophylaxe vorgenommen werden. Als Auswahlkriterien für eine solche Hochrisikogruppe können bspw. die Tatsachen dienen, daß mindestens eine Verwandte ersten Grades mütterlicherseits entweder vor dem 45. Lebensjahr oder beidseitig an Brustkrebs erkrankt ist, oder das mütterlicherseits mindestens eine Verwandte ersten Grades und eine zusätzliche Verwandte an Brustkrebs erkrankt sind. Da die erfindungsgemäße lokale Applikation mögliche systemische Nebenwirkungen des Wirkstoffes aufgrund dessen Hydrophobie praktisch vollständig vermeidet, kann die Indikation zur Primärprophylaxe relativ großzügig bereits bei Vorliegen von Gewebe mit verhältnismäßig geringem oder mittlerem Risiko (bspw. Histologischer Befund Prechtel II oder III) gestellt werden. Mit der Prophylaxe kann begonnen werden, auch wenn die herkömmliche Frühdiagnostik (Palpationsbefund) noch negativ ist. Denn diese übliche Frühdiagnostik ist unzureichend und erkennt ein Mammakarzinom in der Regel erst dann wenn bereits eine kaum noch curable systemische Erkrankung vorliegt.

Das erfindungsgemäße Medikament wird vorzugsweise über einen längeren Zeitraum (bei Bedarf bis lebenslang) lokal und topisch appliziert, die Applikation erfolgt bspw. ein- bis zweimal pro Tag.

Die Wirksubstanzen werden bevorzugt aus der Gruppe der (bevorzugt steroidalen und lipidlöslichen) Aromataseinhibitoren ausgewählt. Diese lipidlöslichen Substanzen dringen bei topischer Applikation in das Fettgewebe ein und behindern lokal die de-novo-Bildung von Östrogenen aus den Östrogenvorstufen.

Verwendbar sind bspw. steroidale Aromataseinhibitoren wie 4-Hydroxyandrost-4-en-3,17-dion (Formestan), 6-Methylen-androstra-1,4-dien-3,17-dion (Exemestan), 10-(2-Propynyl)ester-4-en-3,17-dion (MDL 18962) und 7-α-substituierte Androstendionderivate.

Bei den in Klammern genannten Bezeichnungen handelt es sich um die INN (International nonproprietary names). Zur Terminologie und Struktur der genannten Substanzen wird gleichfalls auf die Rote Liste sowie Römpp Chemielexikon verwiesen.

Die genannten Substanzen sind bisher nur für die systemische Therapie von Brustkrebs verwendet worden. Erfindungsgemäß wird dagegen der Wirkstoff durch lokale Applikation transdermal an den vorgesehenen Wirkort gebracht. Bei der Verwendung von Aromataseinhibitoren erreicht die Erfindung eine Verminderung der Aromataseaktivität im Fettgewebe der Brust, also genau an der Stelle, an der ein Tumor entstehen oder wachsen kann. Bei längerfristiger Anwendung wird die Fettmasse der Brust und damit die Menge an möglichem Risikogewebe reduziert. Da Mammakarzinome häufig in oberen Brustquadranten erhöhter Aromataseaktivität entstehen, ist dort erfindungsgemäß eine besonders wirkungsvolle Prophylaxe möglich.

Die erfindungsgemäß besonderes bevorzugte Verwendung von Aromataseinhibitoren kann prophylaktisch oder therapeutisch auch gegen solche Tumore der Mamma eingesetzt werden, die selbst in der Lage sind, Östrogen für autokrine/parakrine Stimulation zu produzieren. Eine Senkung der Östrogenkonzentration im Plasma wirkt sich auf solche Tumoren kaum aus, die erfindungsgemäß zu erzielende Verminderung der intratumoralen Aromatasekonzentration aufgrund der Verwendung zellgängiger Inhibitoren kann jedoch solche Tumore beeinflussen.

Da die erfindungsgemäß zugeführten Wirkstoffe aufgrund ihrer Lipidlöslichkeit im Fettgewebe der Brust lokalisiert bleiben und dort ihre vorgesehene Wirkung entfalten, scheiden die durch eine systemische Applikation induzierten Nebenwirkungen aus. Diese Verminderung bzw. Ausschaltung von Nebenwirkungen erlaubt eine wesentlich breitere prophylaktische Anwendung. Das erfindungsgemäße Medikament kann von Patientinnen selbst appliziert werden, häufige Arztbesuche zu diesem Zweck sind nicht erforderlich.

Bevorzugt enthält ein erfindungsgemäß formuliertes Medikament Formestan.

Formestanderivate wie bspw. acetyliertes Formestan sind ebenfalls bevorzugt verwendbar.

Die erfindungsgemäß verwendeten Wirkstoffe sind in der Regel lipidlöslich und eignen sich gut für eine transdermale Applikation. Wie oben bereits beschrieben, vermeidet die Anreicherung und kapillare Resorption im Fettgewebe systemische Nebenwirkungen. Zur Erhöhung der transdermalen Resorption können dem erfindungsgemäßen Medikament im Stand der Technik bekannte Stoffe hinzugefügt werden, die diese Resorption fördern, bspw. Hyaluronidate oder DMSO (Dimethylsulfoxid).

Das Medikament ist vorzugsweise als Salbe, Creme, Gel, Emulsion oder Lotion formuliert. Eine Formulierung als Puder oder Öl ist ebenfalls denkbar. Formulierungsgrundlagen sind dem Fachmann aus der kosmetischen und pharmazeutischen Industrie geläufig und brauchen hier nicht näher erläutert zu werden. Verwendbar sind bspw. pflanzliche Öle und Fette wie Mandelöl, Erdnußöl, Olivenöl, Pfirsichkernöl, Rizinus- bzw. Castoröl, Pflanzenextrakte, etherische Öle; ferner pflanzliche Wachse sowie synthetische und tierische Öle, Fette oder Wachse; Lecithin, Lanolinalkohole, Karotin, Duftstoffe, ein- oder mehrwertige Alkohole, Harnstoff, Konservierungs- und Farbstoffe usw. Bevorzugt ist eine Formulierung als Öl-in-Wasser- oder Wasser-in-Öl-Emulsion.

Der Wirkstoffgehalt des Medikaments (der Gehalt von die Bildung von Östrogenen inhibierenden Substanzen) kann zwischen 0,0001 und 20 Gew.-%, vorzugsweise 0,001 und 5 Gew.-%, weiter vorzugsweise 0,1 und 1,5 Gew.-% liegen.

Sofern Stoffe zur Förderung der transdermalen Resorption beigemischt werden, kann deren Gehalt bei der Verwendung von Hyaluronidaten bspw. zwischen 0,01 und 1 Gew.-%, vorzugsweise 0,05 und 0,2 Gew.-% liegen; im Fall der Verwendung von DMSO zwischen 1 und 25 Gew.-%, vorzugsweise 5 und 10 Gew.-%.

Ausführungsformen der Erfindung werden nachfolgend beschrieben. In der Zeichnung zeigen:
- Fig.1: den zytologischen Befund eines Fettzell-Aspirats vor erfindungsgemäßer Anwendung des Medikaments,
- Fig.2: den entsprechenden Befund nach täglicher Anwendung über 3 Monate.

### Beispiel 1

Die folgenden Bestandteile wurden zu einer Creme vermischt:

| | |
|---|---|
| Harnstoff | 10 g |
| Titanoxid | 15 g |
| Rohvaseline | 20 g |
| Isopropylpalmitat | 10 g |
| gehärtetes Erdnußöl | 10 g |
| Tween 80 | 5 g |
| Formestan | 1 g |
| mit gereinigtem Wasser aufgefüllt auf | 100 g |

### Beispiel 2

Ein Gel wurde aus folgenden Bestandteilen hergestellt:

| | |
|---|---|
| Ethanol 90% | 7,0 g |
| Carbopol^{R} 934 P | 7,0 g |
| Triethanolamin | 2 g |
| Polysorbat 80 | 5,0 g |
| Glycerol | 3,0 g |
| Formestan | 0,75 g |
| gereinigtes Wasser auf | 100 g |

### Beispiel 3

Eine Creme wurde aus folgenden Bestandteilen hergestellt:

| | |
|---|---|
| Propylenglykol | 25,0 g |
| Isopropylmyristat | 6,0 g |
| Sorbitanmonostearat | 1,0 g |
| Polysorbat 80 | 2,0 g |
| Cetylstearylalkohol | 6,0 g |
| Stearylalkohol | 2,0 g |
| Glycerolmonostearat | 1,0 g |
| Hyaluronsäure | 0,1 g |
| Formestan | 1,5 g |
| gereinigtes Wasser auf | 100 g |

### Beispiel 4

Eine Creme wurde aus den nachfolgenden Bestandteilen hergestellt. Die Bestandteile sind bei diesem Beispiel mit ihren INCI-Namen angegeben.

| INCI | |
|---|---|
| Ceteareth-25 | 3,0 g |
| PEG-4-Polyglyceryl-2-Stearate | 2,0 g |
| Cetearyl Alcohol | 4,9 g |
| Petrolatum | 10,0 g |
| Paraffinum Perliquidum | 3,0 g |
| Sodium Carbomer 400 | 0,14 g |
| Lactic Acid | 0,02 g |
| Paraffinum Perliquidum | 2,0 g |
| Phenoxyethanol, Dehydroacetic Acid, Benzoic Acid | 0,4 g |
| Parfum | 0,08 g |
| Formestan | 1,5 g |
| aufgefüllt mit Wasser auf | 100 g |

Die in der Formulierung genannte Mischung aus Phenoxyethanol, Dehydroessigsäure und Benzoesäure ist von der Firma Schülke & Mayr unter dem Namen Euxyl^{R} K702 erhältlich.

### Beispiel 5

Ein klinischer Test der Rezeptur gemäß Beispiel 1 wurde durchgeführt. Bei der 25jährigen Probandin lagen die Befunde übergroße Brüste sowie eine mittelgradige Mastopathie vor.

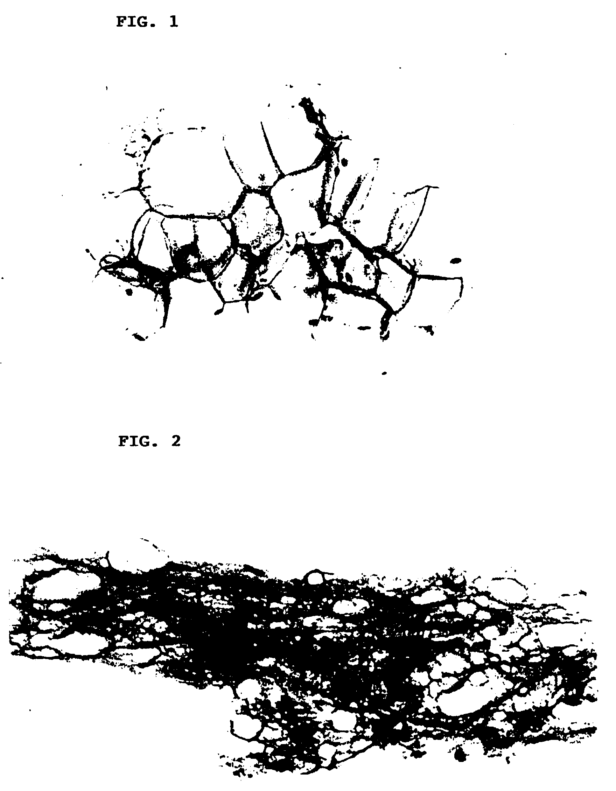

Ein Feinnadel-Aspirat des Fettgewebes (0,6 mm Punktionskanüle, Fixation in absolutem Ethanol, Färbung: May-Grünwald-Giemsa) wurde entnommen (Fig.1). Man erkennt aufgeblähte Fettzellen und exzentrische Zellkerne durch hohen Östrogeneinfluß.

Die Probandin applizierte anschließend die Creme gemäß Beispiel 1 zweimal täglich über einen Zeitraum von 3 Monaten. Fig.2 zeigt das Fettgewebe-Aspirat nach dieser Anwendung. Man erkennt eine durch die Aromatasehemmung bewirkte Volumenreduktion des Fettgewebes ( 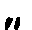 geschrumpfte" Fettzellen in regelmäßiger Anordnung) sowie eine Bindegewebsvermehrung. Der Befund ergab eine Verminderung des Risikogewebes um ca. 50% sowie eine deutliche Straffung von Bindegewebe und Haut.

## Patentansprüche

1. Verwendung einer oder mehrerer die Bildung von Östrogenen inhibierenden Substanzen zur Herstellung eines für die topische Applikation formulierten Medikaments zur Prophylaxe und/oder Behandlung des Mammakarzinoms.

2. Verwendung nach Anspruch 1, dadurch gekennzeichnet, daß das Medikament Substanzen ausgewählt aus der Gruppe der Aromataseinhibitoren enthält.

3. Verwendung nach Anspruch 2, dadurch gekennzeichnet, daß das Medikament Formestan (4-Hydroxyandrost-4-en-3,17-dion) enthält.

4. Verwendung nach einem der Ansprüche 1 bis 3, dadurch gekennzeichnet, daß das Medikament zusätzlich Stoffe zur Förderung der transdermalen Resorption enthält.

5. Verwendung nach Anspruch 4, dadurch gekennzeichnet, daß das Medikament DMSO enthält.

6. Verwendung nach einem der Ansprüche 1 bis 5, dadurch gekennzeichnet, daß das Medikament als Salbe, Creme, Gel, Emulsion oder Lotion formuliert ist.

7. Verwendung nach Anspruch 6, dadurch gekennzeichnet, daß der Wirkstoffgehalt 0,0001-20 Gew.-%, vorzugsweise 0,001-5 Gew.-%, weiter vorzugsweise 0,1-1,5 Gew.-% beträgt.
